(19) Europäisches Patentamt
European Patent Office
Office européen des brevets

(11) Publication number : **0 376 882 B1**

# (12) EUROPEAN PATENT SPECIFICATION

(45) Date of publication of patent specification : **11.08.93 Bulletin 93/32**

(21) Application number : **89810939.2**

(22) Date of filing : **12.12.89**

(51) Int. Cl.$^5$ : **C07C 323/12,** C07C 317/18, C07C 323/25, C07C 311/09, C07C 219/06, D06M 13/252, D06M 13/268, D06M 13/438, D06M 13/372

## (54) Heteroatom containing perfluoroalkyl terminated neopentyl acids and salts thereof.

(30) Priority : **19.12.88 US 286548**

(43) Date of publication of application : **04.07.90 Bulletin 90/27**

(45) Publication of the grant of the patent : **11.08.93 Bulletin 93/32**

(84) Designated Contracting States : **BE CH DE FR GB IT LI NL SE**

(56) References cited :
FR-A- 2 199 536
FR-A- 2 340 307
FR-A- 2 340 930
FR-A- 2 416 222

(73) Proprietor : **CIBA-GEIGY AG**
**Klybeckstrasse 141**
**CH-4002 Basel (CH)**

(72) Inventor : **Falk, Robert A.**
**35 Glenside Drive**
**New City New York 10956 (US)**
Inventor : **Clark, Kirtland P.**
**10 Allan Way**
**Bethel Connecticut 06801 (US)**

EP 0 376 882 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to heteroatom containing perfluoroalkyl terminated neopentyl carboxylic acids and salts thereof and use to impart oil and water repellency to cellulosic, natural and synthetic polyamide, polyester, leather and other compositions.

Bis-perfluoroalkyl terminated neopentyl carboxylic acids devoid of oxygen, sulfur or nitrogen heteroatoms have been described in U.S. Patent Nos. 3,478,116 and 3,578,701. However such compounds are difficult and expensive to prepare and therefore are impractical and not useful products. Additionally, other bis-perfluoroalkyl carboxylic acids are described in U.S. Patent Nos. 4,239,915, 4,419,298, and 4,426,466. These compounds are all thermally unstable and not recommended for ovenable applications, or expensive to prepare and impractical from the standpoint of cost considerations. The bis-perfluoroalkyl carboxylic acids according to the present invention are readily prepared in high yield, are thermally stable, and relatively inexpensive. Another advantage is that the pendant perfluoroalkyl chains are connected by flexible nitrogen, oxygen, or sulfur heteroatoms to the remainder of the molecule thus providing more mobile perfluoroalkyl functions.

Bis-perfluoroalkyl carboxylic acids are useful because they possess a low free surface energy which when applied to a wide variety of substrates provides oil and water repellency. Acids containing a single $R_f$-function are much more readily available but do not provide nearly the same degree of repellency, especially when applied from solution or emulsion to preformed paper. The bis-perfluoroalkyl carboxylic acids according to the present invention may be prepared in high yield and purity in contrast to prior art materials. Most importantly the bis-perfluoroalkyl carboxylic acids of the present invention are thermally stable.

In addition, this invention provides cellulose, leather, or fabrics treated with the bis-perfluoroalkyl carboxylic acids of the present invention and shaped articles made therefrom.

Also, the invention provides a method for treating the aforesaid materials to impart oil and water repellency thereto.

The use of the bis-perfluoroalkyl carboxylic acids according to the present invention provides thermally stable materials which are oil and water repellent at lower application levels than have been provided by previously utilized treating compositions.

One object of the invention are compounds of the formula I

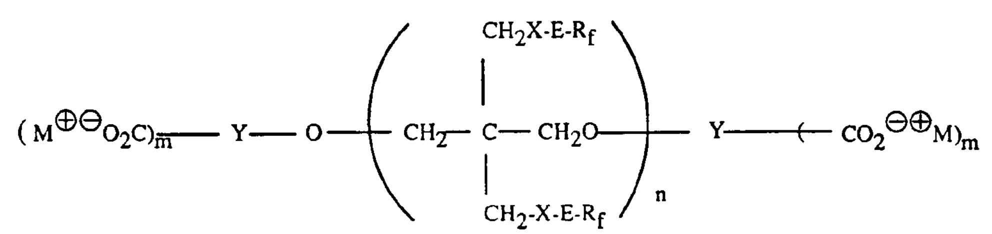

(I),

wherein $R_f$ is a hydrophobic, oleophobic fluoroaliphatic group of up to 20 carbon atoms, E is alkylene of up to 10 carbon atoms, optionally interrupted by up to three groups including -NR-, -O-, -S-, $-SO_2-$, $-O_2C-$, -CONR-, and $-SO_2NR-$, X is -S-, $-SO_2-$, -O-, or -NR-, Y is independently hydrogen when one m means 0, or Y is straight or branched chain alkylene, tri- or tetra-radical of up to 10 carbons, optionally interrupted by -O-, -S-, or -CH=CH-, or carbonylalkylene, arylene, or carbonylarylene, R is independently hydrogen, alkyl, or hydroxyalkyl of 1 to 6 carbon atoms, n is 1 to 3, m is independently 0, 1, 2, or 3 with the proviso that m cannot be 0 in both instances and M is independently hydrogen, an alkali metal, ammonium or organoammonium ion.

It is understood that the $R_f$ group usually represents a mixture of perfluoroalkyl moieties. When the $R_f$ group is identified as having a certain number of carbon atoms, said $R_f$ group also usually concomitantly contains a small fraction of perfluoroalkyl groups with lower carbon atoms and a small fraction of perfluoroalkyl groups with higher carbon content.

A preferred group of compounds of formula I are those, wherein Y is an optionally interrupted alkylene, or Y is linked via a -CO-group to the oxygene atom.

Preferably the instant compounds of formula I are those where $R_f$ is perfluoroalkyl of 2 to 14 carbon atoms or perfluoroalkyl of 2 to 6 carbon atoms substituted by perfluoroalkoxy of 2 to 6 carbon atoms, E is alkylene of 2 to 6 carbon atoms, $-CONHCH_2CH_2-$, $-CH_2CH_2N(CH_3)CH_2CH_2-$, $CH_2CH_2SO_2NCH_2CH_2-$ or $SO_2NCH_2CH_2-$, X is -S- or $-SO_2-$, and m is 1 or 2, M is an organoammonium ion.

$R_f$ is preferably perfluoroalkyl of 2 to 14 carbon atoms, perfluoroalkoxy optionally substituted by of 2 to 6 carbon atoms. More preferably $R_f$ is perfluoroalkyl of 6-14 carbon atoms and most preferably, of 6 to 12 carbon

atoms.

E is preferably a branched or straight chain alkylene of 2-6 carbon atoms, optionally interrupted by one group selected from the group consisting of -NR-, -O-, -S-, $-SO_2-$, $-O_2C-$, -CONR-, and $-SO_2NR-$. Most preferably E is ethylene.

Preferably X is -S-, $-SO_2$, or -O-; most preferably -S-, or $-SO_2-$.

Y is preferably independently hydrogen or a straight or branched chain alkylene, or tri-radical of up to 6 carbon atoms, optionally interrupted by -O-, -S-, or -CH=CH-, or carbonylalkylene, arylene, or carbonylarylene. More preferably, said alkylene is interrupted by carbonylalkylene.

R is independently hydrogen, alkyl of 1 to 7 carbon atoms or hydroxyalkyl of 1-6 carbon atoms. Preferred alkyl groups are those containing 1-7 carbon atoms such as methyl, ethyl and the like; more preferably methyl. Preferred hydroxyalkyl groups are those containing 1-3 carbon atoms, most preferably hydroxyethyl. Most preferably R is hydrogen, methyl or hydroxyethyl.

Preferably n is 1 or 2 and m is independently 1-3, more preferably 1-2 and most preferably 1.

M is independently hydrogen, an alkali metal, ammonium or organoammonium ion. Suitable alkali metals include sodium, potassium and lithium. Preferably, sodium or potassium is the alkali metal. Suitable organoammonium ions include diethanolammonium, triethanolammonium, triethylammonium and the like, preferably triethanolammonium. Preferably M is an organoammonium ion.

Preferred are compounds where $R_f$ is perfluoroalkyl of 6 to 14 carbon atoms, E is an alkylene link of up to 10 carbon atoms, X is sulfur, and Y is a carbonyl-alkylene linkage (ester function), m = 1, and M is an organoammonium ion, i.e.

$MO_2CC_2H_4CO_2CH_2C(CH_2SCH_2CH_2R_f)_2CH_2O_2CC_2H_4CO_2M$

Most preferred are compounds where $R_f$ is perfluoroalkyl of 6 to 12 carbon atoms, E is ethylene, X is $-SO_2-$, Y is an alkylene linkage, m = 1, and M is an organoammonium ion, i.e.

$MO_2CCH_2O[CH_2C(CH_2SO_2CH_2CH_2R_f)_2CH_2O]_2CH_2CO_2M$

The novel carboxylic derivatives can be obtained directly from heteroatom containing $R_f$-terminated neopentyl glycols of the general formula

$HO[CH_2CX\text{-}E\text{-}R_f)_2CH_2O]_{1-3}\text{-}H$ (II), i.e.

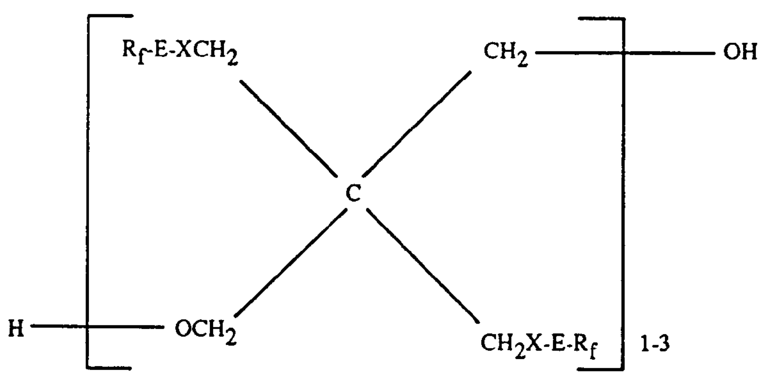

(II)

where

$R_f$ is straight or branched chain perfluoroalkyl of 1 to 18 carbon atoms or said perfluoroalkyl substituted by perfluoroalkoxy of 2 to 6 carbon atoms, or mixtures thereof,

E is branched or straight chain alkylene of up to 10 carbon atoms, optionally interrupted by up to three groups including -NR-, -O-, -S-, $-SO_2-$, $-CO_2-$, $-O_2C-$, -CONR-, and $-SO_2NR-$, X is -S-, $-SO_2-$, -O-, or -NR, where R is independently hydrogen, akyl or hydroxyalkyl of 1 to 6 carbon atoms.

The neopentyl glycols are converted to ether-linked carboxylates by akylation with carboxyl function containing alkyl halides by the classical Williamson synthesis.

Since iodides react more readily than bromides and chlorides are still less reactive, it is recommended to improve the reactivity of the chlorides or bromides by addition of sodium iodide. Haloacids useful for purposes of this invention include:

chloroacetic acid, 6-bromohexanoic acid, 2-bromomethyl acrylic acid, 4-(chloromethyl)benzoic acid, bromosuccinic acid.

Alternately the ether linked carboxylates may be formed by ring-opening alkylation with gamma-butyrolactone or other lactones as described in U.S. 4,720,578. Useful lactones include methyl butyrolactone, dimethyl butyrolactone, ethyl butyrolactone, tributyl butyrolactone, diethyl butyrolactone, triethyl butyrolactone and the like.

This method involves the reaction of a metal hydroxide, such as sodium,potassium or lithium hydroxide to produce the corresponding intermediate salt.

The water generated during the hydroxide condensation reaction can be removed, e.g. by venting to the atmosphere or the water of reaction can be allowed to remain in the system and be recovered with the final product. Alternately, a metal hydride can be used to generate the intermediate salt, typically lithium hydride. The intermediate salt formed by the condensation reaction is then reacted with a butyrolactone. The reactions can be carried out in the presence or absence of an inert solvent such as N-methyl pyrrolidone, chlorobenzene, toluene, xylene, cyclohexane, tetrahydrofuran and the like or an excess of the butyrolactone reactant. The concentration of solvent, when used, is not critical. For example 20-40 % by weight of the active components in the reaction may be used. While the alcohol, inorganic hydroxide and butyrolactone can be intermixed and reacted in a one stage operation, the synthesis can also be carried out in two stages, namely by reacting the alcohol with the inorganic hydroxide in a first stage and then reacting the resulting salt with the butyrolactone in a second stage of the process to produce the bis-perfluoroalkyl carboxylic acids of the present invention. For economic consideration, the mole ratio of alcohol to hydroxide fed to the reaction zone should be close to stoichiometry.

The above reactions can be carried out at temperatures between about 25°C and about 235°C under atmospheric pressure for a period of from about 0.1 to about 50 hours, alternatively higher reaction temperatures under superatmospheric pressure can be employed when desired. Preferably, it is recommended that the reaction be carried out with agitation and at a temperature between about 50° and about 200°C under atmospheric pressure for a period of from about 1 to about 20 hours.

Since the components of the present reaction are non-corrosive, acid resistant equipment is not required and since only a stoichiometric proportion of the alcohol and inorganic hydroxide need be employed the product can be obtained in yields greater than 90 %.

Alternately, the neopentyl glycols can be converted to ester-linked carboxylates by reaction with anhydrides. This reaction can be conducted at 20-100 % solids at about 25°C to about 200°C under atmospheric pressure for a period of about 0.5 to about 50 hours, although higher reaction temperatures can be maintained under superatmospheric pressure when desired. Preferably, the reaction should be carried out with agitation at about 50°C to about 200°C under atmospheric pressure for about 4 to about 20 hours.

Since the anhydride is so reactive, diluents must be chosen on the basis of their inert nature. Suitable diluents may be ethers, such as diethylene glycol dialkyl esters, N-methyl pyrrolidone, esters, such as propylene glycol methyl ether acetate, dimethyl sulfoxide, sulfolane, propylene carbonate, diethyl carbonate, and the like. The reaction is catalyzed by both acids and bases and suitable catalysts are tertiary bases, alkali acetates, zinc chloride, sulfuric acid, dimethylamino-pyridine, a crosslinked polyvinyl pyridine and the like.

Suitable anhydride reactants are cyclic and include halogenated and substituted derivatives of aliphatic, aromatic, alicyclic and heterocyclic acids. The following anhydrides may be used: aconitic, bromomaleic, chloromaleic, citraconic, dichloromaleic, dimethylglutaric, dimethylmaleic, 2-dodecenylsuccinic, 2-ethyl-2-methylsuccinic, glutaric, homophthalic, maleic, itaconic, 2-phenylglutaric, succinic, 5-chloroisatoic, diglycolic, 1,2,4-benzene-tricarboxylic, 3-nitrophthalic, phthalic, trimellic 3,6-endoxo-1,2,3,6-tetrahydrophthalic, isatoic, isocitric anhydride, norbornene, cyclohexane and 2,3-pyridine-dicarboxylic acid anhydride.

Prior-art di-perfluoroalkyl group containing acids as described in U.S. 4,239,915; 4,419,298; 4,426,466; 3,478,116, and 3,578,701 are disclosed as useful, inter alia, for textile treating or as chromium complexes which are useful as grease or oil repellents for paper. Unfortunately, these acids possess, in general, very limited water solubility and therefore require an organic solvent, or the like, for the application of the di-perfluoroalkyl group containing acids to the cellulose, synthetic or natural polyamide substrate in order to obtain the desired oil and water repellent properties. Such organic solvent containing solutions are often undesirable due to odor, toxicity and flammability hazards. Furthermore, such organic solvent containing systems tend to be inefficient.

It is an object of the present invention to avoid such problems by utilizing the ammonium and amine salts of the present invention prepared by reacting the corresponding di-perfluoroalkyl group containing acids with ammonia or the amine in a diluent substantially inert to the acid and ammonia or amine reactants. The reaction can be conducted at temperatures between 0° and about 100°C, preferably at ambient temperature conditions. Where the amine is introduced in gaseous forms, such as anhydrous ammonia or methyl amine, it can be bubbled through the acid in the liquid diluent medium. As the reaction tends to be exothermic, cooling of the reaction vessel may be advantageously employed. Where the inert diluent is organic in nature, such as lower alkanol, for example methanol, diethylene glycol dimethyl ether or the like, the ammonium or amine salts reaction product can be recovered by precipitation, or evaporation of the diluent. The ammonium or amine salt

need not be separated from the solvent media. The amines should be water soluble mono- or polyamines having a water solubility of at least about 2 % by weight. Suitable amines are aminomethane, aminoethane, 1-aminopropane, 2-aminopropane, 1-aminobutane, -amino-2-methyl-propane, 1,1-dimethylethylamine, 1-aminopentane, isoamylamine, tert-amylamine, allylamine, dimethylamine, diethylamine, diisopropylamine, trimethylamine, triethylamine, tri-n-butylamine, ethylenediamine, 1,2-propanediamine, trimethylenediamine, 1,3-diaminobutane, 1,4-diaminobutane, hexamethylene diamine, diethylenetriamine, triethylenetriamine, tetraethylenepentamine, polyethyleneimine having an average of about 20, 80, 120 or 200 units, diethylaminopropylamine, ethanolamine, diethanolamine, triethanolamine, ethylenediaminetetraacetic acid, nitrilotrisacetic acid, N-(hydroxyethyl)-ethylenediamine, N,N"-bis-(hydroxyethyl)-diethylenetriamine, N,N,N',N'-tetrakis-(2-hydroxypropyl)-ethylenediamine, N-(hydroxypropyl)-ethylenediamine, cyclohexylamine, dicyclohexylamine, 1,8-diamino-p-methane, and

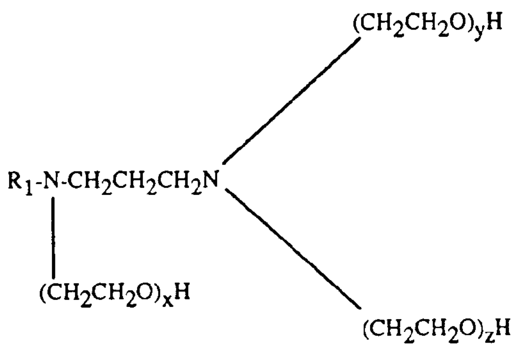

where $R_1$ is tallow fatty alkyl and x+y+z is 3, 10 or 15, fatty diethanolamines, mono-, di, and tri-isopropanolamines and polyoxyethyleneamines.

Alternately, quaternary ammonium salts can be obtained from tetraalkylammonium bases by neutralization.

The bis-perfluoroalkyl carboxylic acids of the present invention can be used in situ by the addition as the free acid to a basic aqueous application formulation. Alternately it is prepared as a concentrate containing about 5 to about 80 % by weight of the neutralized carboxylate salt, optionally in the presence of excess base. The perfluorochemical is applied from water or from a solvent soluble in water to at least 0.1 %.

For topical application, suitable aqueous dilutions contain, advantageously, about 0.01 % to about 5 %, preferably about 0.02 % to about 2 %, by weight of the bis-perfluoroalkyl salts at use dilution. Conventional adjuvants such as water repellant assistants, bacteriostats, coloring agents, surface conditioners and the like, may be included, e.g. in an amount of between about 0.01 % and about 10 % by weight in the emulsion. Also, sizing agents, where the emulsions is to be used on cellulosic substrates, may be present in the amounts of from about 0.01 % to about 10 % by weight.

The sizing agent may be a natural sizing agent such as animal glue, asphalt emulsions, wax emulsions, rosins, starches; a semi-synthetic sizing agent such as a fatty acid salt or complex, a fortified rosin, e.g., tri sodium maleoprimaric acid salt, sodium alginate or sodium carboxymethylcellulose; or a synthetic sizing agent such as an alkylketene dimer, alkylsuccinic anhydrides, polyvinyl alcohol, styrene-maleic anhydride polymers, and the like. Also mixtures thereof may be used.

Additionally, an emulsifier can be optionally present in an amount of between about 0.001 % to about 3 % by weight.

Thus, suitable dilutions for topical application contain;

(a) about 0.01 to about 5 % by weight of the bis-perfluoroalkyl carboxylic acids of the instant invention;

(b) 0 to about 3 % by weight emulsifier;

(c) 0 to about 5 % water repellant assistant, filler, fungicide, bacteriostat, coloring agent or surface conditioner adjuvant;

(d) 0 to bout 10 % sizing agent, and

(e) the remainder water.

These formulations are applied to the surface of the cellulosic or natural or synthetic material by conventional techniques, including padding, spraying, coating, washing, and brushing. After application, the treated surface is dried, with or without an intermediate washing stage. The resulting surface is thus rendered water and oil resistant.

For use as a sizing agent to obtain oil and water repellency, the dilution of the instant aqueous formulations advantageously contain from about 0.0005 to about 0.2 % by weight of the bis-perfluoroalkyl carboxylates. The formulations for dilution may be prepared as a concentrate containing between about 5 % and about 80 % by weight, preferably about 30 to about 80 % by weight, of the salt.

Suitable cellulosic and natural substrates for topical application include paper, non-wowen fabrics, textiles, paperboard, wood, wood fiber products such as plywood, hair, including wool, hides, leather, and feathers. Synthetic substrates include nylon fibers and textiles.

While the instant formulations are suitable for rendering a variety of materials oil and water repellant, they are particularly advantageous in rendering articles made from paper pulp, such as paper trays, paper plates and analogous paper articles, both oleophobic and hydrophobic.

In order to further increase the efficiency of application it is conventional to treat the paper pulp with a cationic agent, or retention aid such as a cationically modified starch, which is adsorbed by the paper pulp and, consequently, tends to increase the amount of fluorochemical transferred to the cellulose substrate.

Suitable cationic agents, conventionally used to treat cellulose materials such as paper pulp, include conventional cationic modified starches, such as Lok-Size 30, Inter-bond C from A.E. Staley; Cato 2, Cato 15 and Cato 17 from Nat. Starch, cationic modified aminoplast resins such as Kymene 557H from Hercules Inc.; cationic polymers such as Hypo WB-4000 from W.R. Grace Inc.

Suitable cationic resins are described in Bates, "Polyamide-Epichlorohydrin Wet Strength Resin, TAPPI, 52, (6) 1969 and in U.S. Patent Nos. 3,655,506 and 4,299,654.

Jointly with the bis-perfluoroalkyl carboxylic acids of the product invention, can be added one or more of a wide choice of water proofing sizing agents selected from classes such as alkyl anhydrides, e.g. Fibron 68 from Nat. Starch; alkyl ketene dimers e.g. Aquapel 360 XC or Hercon 40 from Hercules, Inc; polyurethane emulsions, e.g. acrylic resins, e.g. stearyl amine surfactants, e.g., Ethomeen 18/25 from Akzo Chemie complexed with a fatty acid, e.g. stearic acid; Neofat 14, Neofat 47 or Hystrene 9718 from Akzo Chemie. Suitable hydrophobic sizing agents are described by Davis, et al., TAPPI, 39(1) pp 21-23 (1956) and in U.S. Patent Nos. 4,243,481 and 4,279,794.

The amount of adjuvant and sizing agent used for treating paper is of the range specified for topical applications, supra.

Thus, for internal or external sizing of paper pulp suitable aqueous dilutions contain:

(a) about 0.0005 % to about 0.1 % by weight of the bis-perfluoroalkyl carboxylic acids of the present invention;

(b) 0 to about 0.05 % by weight emulsifier;

(c) 0 to about 5 % by weight filler, bacteriostat, fungicide, coloring agent, surface conditioner adjuvant, or retention aid.

(d) 0 to bout 10 % sizing agent; and

(e) the remainder water.

The following examples illustrates the invention. All parts are by weight unless otherwise specified.

## SAMPLE PREPARATION AND TESTING

### Pad Application of Fluorochemicals on an External Size

Samples of fluorochemicals are diluted to the test application levels with distilled water. The solutions are added to a 4 % aqueous solution of paper maker's starch and applied to unsized paper by padding (paper dipped through starch solution, then passed through single nip rollers). The resulting sheets are dried at ambient conditions for 15 minutes, then 3 minutes at 93,3°C in an "Emerson Speed Drier" (heated metal plate with canvas cover).

### Internal Size Application and Testing

Six grams of dry pulp are diluted in 289 ml distilled water and thoroughly dispersed in a blender. To this pulp slurry is added a 1 % dilution (as is) of the test dispersion in distilled water. This is mixed for 5 minutes, then 6 ml of a 1 % aqueous solution of cooked cationic starch is added. This is mixed togther for an additional 5 minutes. To this, 24 ml of a 50 % (on solids) dilution of a water repellent adjuvant is added. This is mixed in for another 10 minutes. The resulting slurry is diluted with an additional 500 ml of distilled water and mixed again. This mixture is then poured over a 100 mesh wire screen, with a vacuum applied from below which pulls the water from the pulp mixture to form a sheet on the screen. The wet sheet is removed from the screen and dried between another screen and hard surface at a pressure of approximately 27,6 mbar at 110°C for 1 ½

hours. One ml of hot (110°C) corn oil is placed on the paper and the time is noted for penetrations to occur (20 min. max). Similarly, 1 ml of hot (80°C) water containing 0.5 % of Triton X-165 wetting agent (from Rohm & Haas) placed on the paper is tested. Paper made in the same manner, including the cationic starch and water repellant adjuvant but without a fluorochemical demonstrated as oil kit number of <1, and held the hot corn oil and hot water/Tritlon X-165 solution for less than one minute (began to penetrate as soon as applied).

The AATCC Oil Rating is determined according to Standard Test method 118-1983 of the American Association of Textile chemists and Colorists. Ratings are given from 0 (minimum) to 8 (maximim). A commonly accepted level of repellency for oil repellent fabrics in the United States is an oil repellency of 4.

## Grease Resistance Test

Creased test papers are placed over a grid sheet imprinted with 100 squares. 5 grams of sand is placed in the center of the crease. A mixture of synthetic oil and a dye for visualization is pipetted onto the sand and the samples are maintained at 60°C for 24 hours.

Evaluation is determined by the percentage of the grid which is stained.

## Baking Test

A sample is internally sized and heated to 204°C for 30 minutes. The sample is then tested for Oil Kit, and hold-out on hot, 110°C, Corn Oil and hot, 80°C, Triton X-165 solution.

## Example 1:

$$(C_8F_{17}CH_2CH_2SCH_2)_2C(CH_2O_2CCH_2CH_2CO_2H)_2$$

2,2-Bis-(1,1,2,2-tetrahydroperfluorodecylthiomethylene)-1,3-propanediol (15.0 g, 14 mmol) and succinic anhydride (3.45 g, 34 mmol) are reacted under nitrogen with triethylamine (1.0 g, 10 mmol) and dimethylaminopyridine (0.1 g, 0.7 mmol) in 35 g of propylene glycol methyl ether acetate as the solvent. The reaction is run for 3 hours at 90°C, acidified with 20 ml. glacial acetic acid, heated until clear, and slowly precipitated from 700 ml water. The solids are filtered, washed with additional water and dried - m.p. 89-96° by DSC.

Derivatization with bis-(trimethylsilyl)trifluoroacetamide (containing 1 % trimethylchlorosilane) is used for GLC (3 % OV-3 column). The product assayed at 91 % diadduct/3.2 % monoadduct; Equiv. wt. by titration - 643 (627 diadduct calc.); F-50.7 % (51 % diadduct calc.). TGA scans are run at 10°/min., 100 ml $N_2$/min, using a duPont 951 TGA module. There is evidence for decomposition at 140-250°C; more rapidly in air than in nitrogen.

Since several crystallizations from carbon tetrachloride and toluene still do not afford a pure diadduct, purification is accomplished by preparative chromatography on a silica column, eluted by ethyl acetate/methylene chloride/methanol (70/25/5). NMR shows proton resonances at 2.40 ppm, 4 protons, ($CH_2CF_2$); 2.66 ppm, 8 protons; ($CH_2CO_2H$,$\underline{CH_2}CO_2CH_2$); 2.75 ppm, 4 protons, $SCH_2C$; 2.79 ppm, 4 protons, $CH_2\underline{CH_2}S$; 4.30 ppm, 4 protons, $\underline{CH_2}O_2CH_2$.

(Diadduct) MS: m/z 1404 ($M^+$.), 1389 (-$CH_3$), 1215, 957, 173.

(Monoadduct) MS: m/z 1304 ($M^+$.), 1289 (-$CH_3$), 1215, 857, 825.

## Example 2:

$$(C_6F_{13}CH_2CH_2SCH_2)_2C(CH_2OH)_n(CH_2O_2CCH_2CH_2CO_2NHEt_3)_m$$

where n = 0 or 1, m = 1 or 2, and n+m = 2

2,2-Bis-(1,1,2,2-tetrahydroperfluoro-octylthiomethylene)-1,3-propanediol (30.0 g, 35 mmol) and succinic anhydride (7.3 g, 73 mmol) are reacted under nitrogen in triethylamine (9.8 g, 96 mmol) for 2 ½ hours at 80°C (partial conversion to the ester-acid is demonstrated by glc:monoacid-25 %, diacid-62 %). The product is dissolved in 80 g water, 19 g butyl carbitol. The clear solution of triethylamonnium salt has a pH of 8.2 and is at 29 % solids.

Surface tension:dynes/cm in distilled water (% Solids):
19 (0.1); 21 (0.01); 70 (0.001)

## Example 3:

$$(C_8F_{17}CH_2CH_2SO_2CH_2)_2C(CH_2O_2CCH_2CH_2CO_2H)_2$$

2,2-Bis-(1,1,2,2-tetrahydroperfluorodecylthiomethylene)-1,3-propanedioxycarbonylpropionic acid (8.0 g, 6.3 mmol) is dissolved in acetic acid (56.6 g) and the solution sodium perborate tetrahydrate is gradually added (16.3 g, 51 mmol). The exotherm is initially kept below 40°C for 1 hour, and then permitted to rise gradually to 50°C for 2 additional hours. The product is then precipitated in 300 ml of water, filtered, dried, and then crystallized from methyl propyl ketone to yield a white crystalline solid, m.p. 181.5-183.5°C.

Derivatization with bis-(trimethylsilyl)trifluoroacetamide (containing 1 % trimethylchlorosilane) is done for GLC (3 % UV-3 column). The product assayed at 91 % purity.

TGA scans are run at 10°/min., 100 ml $N_2$/min, using a duPont 951 TGA module. There is evidence for decomposition at 160-425°C.

NMR shows proton resonances at 2.62 ppm, 8 protons,

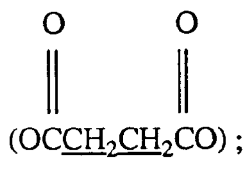

2.8 ppm, 4 protons, ($CF_2\underline{CH_2}$); 3.69 ppm, 4 protons,

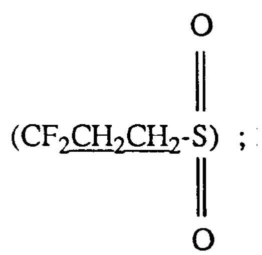

3.99 ppm, 4 protons,

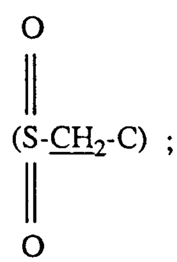

4.47 ppm, 4 proton

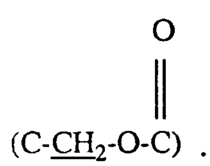

| | | | | |
|---|---|---|---|---|
| Analysis for | $C_{33}H_{26}F_{34}O_{12}S_2$: | | | |
| Calculated: | C, 29.9; | H, 2.0; | F, 48.8; | S, 4.8. |
| Found: | C, 29.8; | H, 1.9; | F, 48.9; | S, 5.5. |

Example 4:

$$(C_8F_{17}CH_2CH_2SCH_2)_2C(CH_2OH)_n(CH_2O_2CH_2CH_2CH_2CO_2H)_m$$

where n = 0 or 1, m = 1 or 2, and n+m = 2

2,2-Bis-(1,1,2,2-tetrahydroperfluorodecylthiomethylene)-1,3-propanediol (15.0 g, 14 mmol) and glutaric anhydride (4.1 g, 36 mmol) are reacted under nitrogen with dimethylaminopyridine (0.2 g, 1.4 mmol), and 2-pentanone (30 g) as the solvent. The reaction is run at 85°C for 11 hours to yield a mixture of mono- and di-adducts. The product is isolated by precipitation, washed with acetone and submitted for identification.

Identification is made by GC/MS (EI mode) after derivatization with N,O-bis-(trimethylsilyl)trifluoroacetamide. Two major components are observed.

(Diadduct)

MS: m/z 1432 ($M^+$.), 1417 ($M-CH_3$), 1413 (M-F), 1228, 985, 493, 187 (b.p.).

(Monoadduct)

MS: m/z 1318 ($M^+$.), 1303 ($M-CH_3$), 1299 (M-F), 1115, 871, 493, 187, 143.

Example 5:

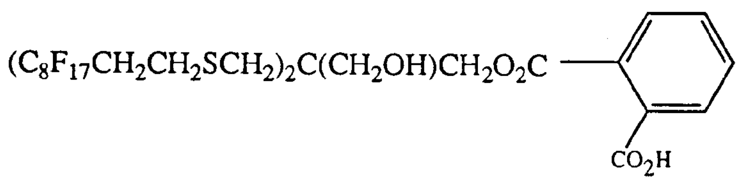

2,2-Bis-(1,1,2,2-tetrahydroperfluorodecylthiomethylene)-1,3-propanediol (4.3 g, 4.1 mmol) and phthalic anhydride (1.5 g, 10.3 mmol) are reacted under nitrogen with dimethylaminopyridine (0.1 g, 0.7 mmol), and 2-pentanone (10 g) as a solvent. The reaction is run at 80°C for 2.5 hours to yield the mono-adduct. The product is isolated by precipitation from water, washed with acetone and methylene chloride, and analyzed.

Identification is made by GC/MS (EI mode) as in Example 4. One major component is observed.

(Monoadduct)

MS: m/z 1352 ($M^+$.), 1337 ($M-CH_3$), 1333 (M-F), 1115, 905, 873, 221, 143, 103.

Example 6:

$$(C_8F_{17}CH_2CH_2SCH_2)_2C(CH_2OH)CH_2O_2CCH{=}CHCO_2H$$

2,2-Bis-(1,1,2,2-tetrahydroperfluorodecylthiomethylene)-1,3-propanediol (15 g, 14.2 mmol) and maleic anhydride (3.9 g, 40 mmol) are reacted under nitrogen with dimethylaminopyridine (0.2 g, 1.4 mmol), and 2-pentanone (30 g) as a solvent. The reaction is run at 85°C for 12 hours to yield the mono-adduct (brown product). The product is isolated by precipitation from 400 ml water with 1 g acetic acid (50 %) and vacuum filtered. A tan product of 19 g results.

Identification is made by GC/MS (EI mode) as in Example 4. One major component is observed.

(Monoadduct) - two isomers

MS: m/z 1302 ($M^+$.), 1287 ($M-CH_3$), 1115, 855.

Example 7:

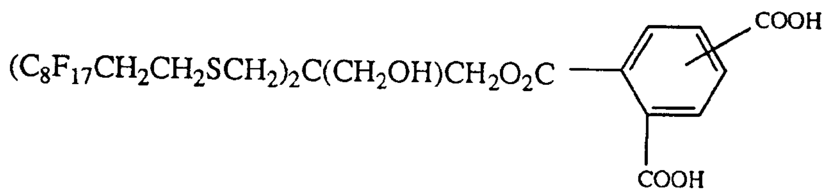

2,2-Bis-(1,1,2,2-tetrahydroperfluorodecylthiomethylene)-1,3-propanediol (15.0 g, 14 mmol) and triamellitic anhydride (7.7 g, 4 mmol) are reacted under nitrogen with dimethylaminopyridine (0.2 g, 0.6 mmol) and 2-pentanone (30 g) as a solvent. The reaction is run at 85°C for 17 hours to yield the mono-adduct. The product is isolated by precipitation from 600 ml water with 2 g acetic acid (50 %) and vacuum filtered.

Identification is made by GC/MS (EI mode) as in Example 4. One major component is observed.

(Monoadduct)

MS: m/z 1468 ($M^+$.), 1453 ($M-CH_3$), 1115, 1021, 143.

Example 8:

$$(C_8F_{17}CH_2CH_2SCH_2)_2C(CH_2OH)_n(CH_2O_2CH_2CH_2CH_2CO_2H)_m$$

where n = 0 or 1, m = 1 or 2, and n+m = 2

2,2-Bis-(1,1,2,2-tetrahydroperfluorodecylthiomethylene)-1,3-propanediol (15.0 g, 14.3 mmol) and itaconic anhydride (4.0 g, 35.8 mmol) are reacted under nitrogen with dimethylaminopyridine (0.2 g, 1.6 mmol), and propylene glycol methyl ether acetate (35 g) as the solvent. The reaction is run at 60°C for 2 hours and at 90°C for 16 hours to yield a mixture of mono- and di-adducts. The product is acidified with acetic acid, isolated by precipitation with 550 g water and vacuum filtered.

Identification is made by GC/MS (EI mode) as in Example 4. Two major components are observed.

(Monoadduct)

MS: m/z 1316 ($M^+$.), 1301 ($M-CH_3$), 1287 (M-F), 1115, 869, 103, 73.

Example 9:

$$(C_8F_{17}CH_2CH_2SCH_2)_2C(CH_2OH)_n(CH_2OCH_2CO_2H)_m$$

where n = 0 or 1, m = 1 or 2, and n+m = 2

2,2-Bis-(1,1,2,2-tetrahydroperfluorodecylthiomethylene)-1,3-propanediol (20.0 g, 19 mmol) and chloroacetic acid (3.6 g, 38.1 mmol) are reacted under nitrogen with potassium carbonate, anhydrous (21.0 g), sodium iodide catalyst (1.0 g, 6.7 mmol), and 2-pentanone (25 g) as the solvent. The reaction is refluxed for 76°C for 18 hours to yield a mixture of mono- and di-adducts. The product is acidified, isolated by precipitation from water and vacuum filtered.

Identification is made by GC/MS (EI mode) as in Example 4. Two major components are observed.

(Monoadduct)

MS: m/z 1262 ($M^+$.), 1247 ($M-CH_3$), 1243 (M-F), 1115, 815, 147, 103.

(Diadduct) - 2 isomers

MS: m/z 1320 ($M^+$.), 1301 ($M-CH_3$), 1173, 873.

Using the methods described and by techniques similar to Examples 1-9, the following sulfido-, sulfone and amine carboxylates can be prepared.

Perfluoroalkyl Terminated Neopentyl Carboxylates

$(CF_3CF_2SCH_2)_2C(CH_2OH)_n(CH_2O_2CH_2CH_2CO_2H)_m$
$(C_6F_{13}(CH_2)_4SO_2CH_2)C(CH_2OH)_n(CH_2O(CH_2)_3CO_2H)_m$
$(C_8F_{17}CH_2CH_2CH_2SCH_2)_2C(CH_2OH)_n(CH_2OCH_2CO_2H)_m$
$(C_8F_{17}CH_2CH_2N(CH_3)CH_2CH_2CH_2SCH_2)_2C(CH_2OH)_n(CH_2OCH_2CO_2H)_m$

$(C_8F_{17}SO_2NHCH_2SCH_2)_2C(CH_2OH)_n(CH_2O_2$ 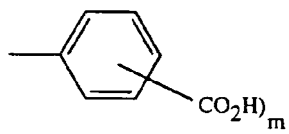

$C_8F_{17}CH_2SO_2NHCH_2CH_2CH_2)_2C(CH_2OH)_n(CH_2O_2CH_2CH_2CO_2H)_m$
$[C_7F_{15}CONH(CH_2)_2SCH_2]_2C(CH_2OH)_n(CH_2OCH_2CO_2H)_m$
$HO_2CCH_2O[CH_2C(CH_2SO_2CH_2CH_2C_8F_{17})_2CH_2O]_2CH_2CO_2H$
$(C_8F_{17}CH_2CH_2NCH_3CH_2)C(CH_2OH)_n(CH_2O_2CCH_2CO_2H)_m$
Reaction product of $(R_fCH_2CH_2SCH_2)_2C(CH_2OH)_2$ and Σ-caprolactone with $ClCH_2CO_2H$ where n = 0 or 1, m = 1 or 2 and n+m = 2.

Example 10: Stability of Bis-perfluoroalkylthio-Diacids

Thermogravimetric analyses are run on the diacids of Examples 1 and 3.
The instrument is run at 10°C/minute to 500°C with 100 ml/min $N_2$ or in air.

TGA (Weight Loss)

Temperature (°C) of Indicated Weight Loss

Under Nitrogen

| Diacid of Example | DSC (Tm/°C) | Initial | 10 % | 50 % |
|---|---|---|---|---|
| 1 | 80-90 | 150 | 285 | 370 |
| 3 | 181-183 | 160 | 230 | 340 |

The data indicate that the bis-perfluoroalkyl carboxylic-acids of the present invention do not appreciably decompose the well over 300°C in content.

Example 11:

This example describes internal size performance evalutions of the subject carboxylic acids, examples 1 and 3. The bis-perfluoroalkyl carboxylic acids of the present invention are prepared by dissolving them in water as triethanolamine salts and using a 200 mol % excess of triethanolamine.

Hold-out Tests

| | Fluorine on weight of paper | Oil Kit Number | Hot, 110°C Corn Oil | Hot, 80°C Water + 0.5 % Triton X-165 |
|---|---|---|---|---|
| Example 1 | 0.04 | 2 | 15 min. | >20 min. |
| | 0.05 | 3-4 | >20 min. | >20 min. |
| | 0.06 | 4 | >20 min. | >20 min. |
| Example 3 | 0.04 | 2 | 3 | >20 min. |
| | 0.05 | 3 | >20 | >20 min. |
| | 0.06 | 3 | >20 | >20 min. |

Example 12:

This example describes external size performance of the bis-perfluoroalkyl carboxylic acids of the present invention examples 1 and 3. The products are applied to paper by pad application and tested for Oil Kit Rating and the Grease Resistance Test.

The results indicate that the bis-perfluoroalkyl carboxylic acids of the present invention have superior peformance at much lower application levels, by the Grease Resistance Test. Further, the novel carboxylates pass the Grease Resistance Test at lower Kit Numbers. This allows their application to products requiring better adhesive bonding and better label adhesion. It further permits the manufacturer to have lessened problems with printing.

| | % Fluorine on Weight of paper | Oil Kit Number | Grease Resistance Test |
|---|---|---|---|
| Example 1 | 0.04 | 5 | pass |
| | 0.05 | 7-8 | pass |
| Example 3 | 0.04 | 2 | fail |
| | 0.05 | 3-4 | pass |

Example 13:

This example describes Baking Test performance of internally sized paper using the bis-perfluoroalkyl carboxylic acids of the present invention examples 1 and 3. The products are applied as in Example 11 at 0.1 % Fluorine on Weight of paper.

The results for the bis-pefluoroalkyl carboxylic acids of the present invention indicate that they have good heat resistance with regard to oil-hold out, but superior performance with regard to water-holdout.

Hold-out Tests

| | Time (min) | Number | Oil Kit Corn Oil | Hot, 110°C Hot, 80°C Water + 0.5 % Triton X-165 |
|---|---|---|---|---|
| Example 1 | 30 | 3 | >20 | >20 |
| Example 3 | 30 | 4 | >20 | >20 |

## Claims

1. Compounds of the formula I

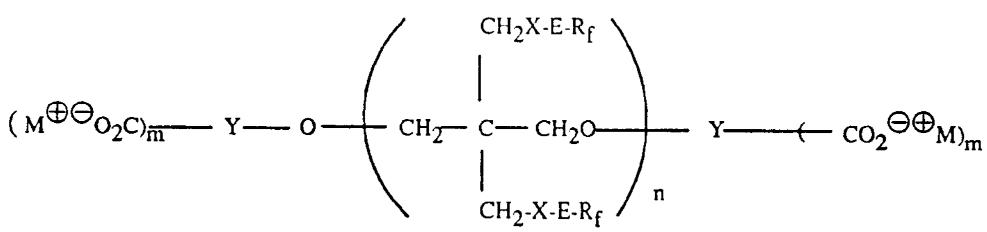

(I),

wherein
$R_f$ is a hydrophobic, oleophobic fluoroaliphatic group of up to 20 carbon atoms;
E is alkylene of up to 10 carbon atoms, optionally interrupted by up to three groups including -NR-, -O-, -S-, $-SO_2-$, $-O_2C-$, -CONR-, and $-SO_2NR-$; X is -S-, $-SO_2-$, -O-, or -NR-;
Y is independently hydrogen when one m is 0, or Y is straight or branched chain alkylene, tri- or tetra-radical of up to 10 carbon atoms or a straight or branched chain alkylene, tri- or tetra-radical of up to 10 carbon atoms interrupted by by a group selected from the group consisting of -O-, -S-, or -CH=CH-, or carbonyl-alkylene, arylene, or carbonylarylene;
R is independently hydrogen, alkyl, or hydroxyalkyl of 1 to 6 carbon atoms;
n is an integer from 1 to 3;
m is independently 0, 1, 2, or 3 with the proviso that m cannot be 0 in both instances;
M is independently hydrogen, an alkali metal, ammonium or organoammonium ion.

2. A compound according to claim 1 wherein said $R_f$ is a perfluoroalkyl of 2 to 14 carbon atoms or a perfluoroalkyl of 2 to 6 carbon atoms substituted by perfluoroalkyl of 2 to 6 carbon atoms.

3. A compound according to claim 1 wherein said $R_f$ is a perfluoroalkyl of 6-14 carbon atoms;

4. A compound according to claim 1 wherein said E is a branched or straight chain alkylene of 2-6 carbon atoms, optionally interrupted by one group selected from the group consisting of -NR-, -O-, -S-, $-SO_2-$, $-O_2C-$, -CONR-, and $-SO_2NR-$.

5. A compound according to claim 1 wherein said E is ethylene.

6. A compound according to claim 1 wherein said X is -S-, $-SO_2$, or -O-.

7. A compound according to claim 1 wherein said X is -S-, or $-SO_2-$.

8. A compound according to claim 1 wherein said Y is independently hydrogen or a straight or branched chain alkylene, or tri-radical of up to 6 carbon atoms interrupted by -O-, -S-, or -CH=CH-, carbonylalkylene, arylene, or carbonylarylene.

9. A compound according to claim 8 wherein said alkylene, or tri-radical is interrupted by carbonylalkylene.

10. A compound according to claim 1 wherein said R is independently hydrogen, alkyl of 1-7 carbon atoms or hydroxyalkyl of 1-6 carbon atoms.

11. A compound according to claim 1 wherein said R is independently hydrogen, methyl or hydroxyethyl.

12. A compound according to claim 1 wherein said n is 1 or 2 and m is independently 1-3.

13. A compound according to claim 1 wherein said M is an organoammonium ion.

14. A compound according to claim 1 wherein said $R_f$ is a perfluoroalkyl of 6 to 14 carbon atoms; E is an alkylene link of up to 10 carbon atoms, X is -S-; Y is a carbonyl-alkylene; m = 1 and M is an organoammonium ion.

15. A compound according to claim 1 wherein said $R_f$ is perfluoroalkyl of 6 to 12 carbon atoms; E is ethylene; X is $-SO_2-$; Y is an alkylene; m = 1 and M is an organoammonium ion.

16. A compound according to claim 1 wherein said $R_f$ is perfluoroalkyl of 2 to 14 carbon atoms or perfluoroalkyl of 2 to 6 carbon atoms substituted by perfluoroalkoxy of 2 to 6 carbon atoms, E is alkylene of 2 to 6 carbon atoms, $-CONHCH_2CH_2-$, $-CH_2CH_2N(CH_3)CH_2CH_2-$, $-CH_2CH_2SO_2NCH_2CH_2-$ or $-SO_2NCH_2CH_2-$; X is -S- or $-SO_2-$; m is 1 or 2 and M is an organoammonium ion.

17. A sizing treatment composition comprising at least one compound according to claim 1.

18. Sizing treatment compositions comprising about 0.0005 to about 0.2 % by weight of at least one compound according to claim 1; 0 to about 0.05 % by weight of emulsifier; 0 to about 5 % by weight repellant assistant, fungicide, filler, bacteriostat, coloring agent or surface conditioner adjuvant; 0 to about 10 % sizing agent and the remainder water.

19. The use of compounds of formula I according to claim 1 for the treatment of surfaces to impart oil and water repellency.

## Patentansprüche

1. Verbindungen der Formel I

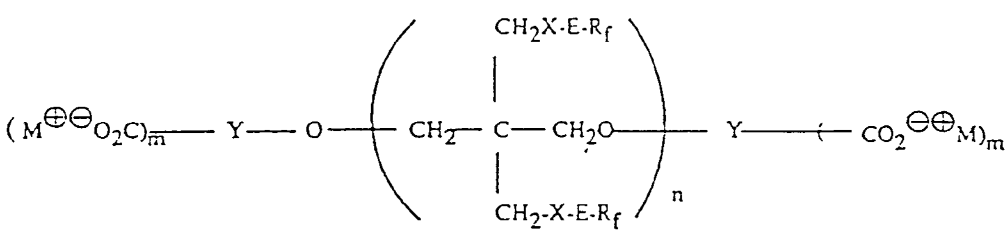

(I)

worin bedeuten:

$R_f$ eine hydrophobe, oleophobe fluoraliphatische Gruppe mit bis zu 20 Kohlenstoffatomen;

E Alkylen mit bis zu 10 Kohlenstoffatomen, welches wahlweise durch bis zu drei Gruppen, einschließlich -NR-, -O-, -S-, $-SO_2-$, $-O_2C-$, -CONR- und $-SO_2NR-$ unterbrochen ist;

X -S-, $-SO_2-$, -O- oder -NR-;

Y unabhängig Wasserstoff, wenn ein m 0 ist oder Y ist ein gerad- oder verzweigtkettiges Alkylen, drei- odervierwertiger Rest mit bis zu 10 Kohlenstoffatomen oder ein gerad- oder verzweigtkettiges Alkylen, drei- oder vierwertiger Rest mit bis zu 10 Kohlenstoffatomen, unterbrochen durch eine Gruppe, welche aus der aus -O-, -S- oder -CH=CH- bestehenden Gruppe gewählt ist, oder Carbonylalkylen, Arylen oder Carbonylarylen;

R unabhängig Wasserstoff, Alkyl oder Hydroxyalkyl mit 1 bis 6 Kohlenstoffatomen;
n eine ganze Zahl von 1 bis 3;
m unabhängig 0, 1, 2 oder 3, mit der Maßgabe, daß m nicht in beiden Fällen 0 sein kann;
M unabhängig Wasserstoff, ein Alkalimetall, Ammonium- oder Organoammoniumion.

**2.** Verbindung nach Anspruch 1, worin $R_f$ ein Perifluoralkyl mit 2 bis 14 Kohlenstoffatomen oder ein durch Perfluoralkyl mit 2 bis 6 Kohlenstoffatomen substituiertes Perfluoralkoxy mit 2 bis 6 Kohlenstoffatomen ist.

**3.** Verbindung nach Anspruch 1, worin $R_f$ ein Perfluoralkyl mit 6 bis 14 Kohlenstoffatomen ist.

**4.** Verbindung nach Anspruch 1, worin E ein verzweigt- oder geradkettiges Alkylen mit 2 bis 6 Kohlenstoffatomen ist, welches wahlweise durch eine Gruppe unterbrochen ist, welche aus der aus -NR-, -O-, -S-, $-SO_2-$, $-O_2C-$, -CONR-, und $-SO_2NR-$ bestehenden Gruppe gewählt ist.

**5.** Verbindung nach Anspruch 1, worin E Ethylen ist.

**6.** Verbindung nach Anspruch 1, worin X die Bedeutung -S-, - $SO_2$ - oder -O- hat.

**7.** Verbindung nach Anspruch 1, worin X die Bedeutung -S- oder $-SO_2-$ hat.

**8.** Verbindung nach Anspruch 1, worin Y unabhängig Wasserstoff oder ein gerad- oder verzweigtkettiges Alkylen oder ein dreiwertiger Rest mit bis zu 6 Kohlenstoffatomen, unterbrochen durch -O-, -S- oder -CH=CH-, Carbonylalkylen, Arylen oder Carbonylarylen ist.

**9.** Verbindung nach Anspruch 8, worin das Alkylen oder der dreiwertige Rest durch Carbonylalkylen unterbrochen ist.

**10.** Verbindung nach Anspruch 1, worin R unabhängig Wasserstoff, Alkyl mit 1 bis 7 Kohlenstoffatomen oder Hydroxyalkyl mit 1 bis 6 Kohlenstoffatomen ist.

**11.** Verbindung nach Anspruch 1, worin R unabhängig Wasserstoff, Methyl oder Hydroxyethyl ist.

**12.** Verbindung nach Anspruch 1, worin n 1 oder 2 ist und m unabhängig 1 bis 3 ist.

**13.** Verbindung nach Anspruch 1, worin M ein Organoammoniumion ist.

**14.** Verbindung nach Anspruch 1, worin $R_f$ einPerfluoralkyl mit 6 bis 14 Kohlenstoffatomen ist; E ein Alkylenverbindungsglied mit bis zu 10 Kohlenstoffatomen ist, X -S- ist; Y ein Carbonylalkylen ist; m = 1 und M ein Organoammoniumion ist.

**15.** Verbindung nach Anspruch 1, worin $R_f$ ein Pefluoralkyl mit 6 bis 12 Kohlenstoffatomen ist; E Ethylen ist; X $-SO_2-$ ist; Y ein Alkylen ist; m = 1 und M ein Organoammoniumion ist.

**16.** Verbindung nach Anspruch 1, worin $R_f$ ein Pefluoralkyl mit 2 bis 14 Kohlenstoffatomen oder ein durch Perfluoralkoxy mit 2 bis 6 Kohlenstoffatomen substituiertes Perfluoralkyl mit 2 bis 6 Kohlenstoffatomen ist; E Alkylen mit 2 bis 6 Kohlenstoffatomen, $-CONHCH_2\ CH_2-$, $-CH_2CH_2N(CH_3)\ CH_2CH_2-$, $-CH_2CH_2SO_2NCH_2CH_2-$ oder $-SO_2NCH_2CH_2-$ ist; X -S- oder $-SO_2-$ ist; m 1 oder 2 ist und M ein Organoammoniumion ist.

**17.** Schlichte-Behandlungszusammensetzung, umfassend mindestens eine Verbindung gemäß Anspruch 1.

**18.** Schlichte-Behandlungszusammensetzungen, umfassend etwa 0,0005 bis etwa 0,2 Gew.-% mindestens einer Verbindung gemäß Anspruch 1; 0 bis etwa 0,05 Gew.-% eines Emulgiermittels; 0 bis etwa 5 Gew.-% eines Abstoßungshilfsmittels, Fungizids, Füllstoffs, Bakteriostats, Färbemittels oder Oberflächenkonditionierhilfsmittels; 0 bis etwa 10 % Schlichtemittel und den Rest Wasser.

**19.** Verwendung von Verbindungen der Formel I gemäß Anspruch 1 zur Behandlung von Oberflächen, um Öl- und Wasserabstoßung zu verleihen.

## Revendications

1. Composés de formule I :

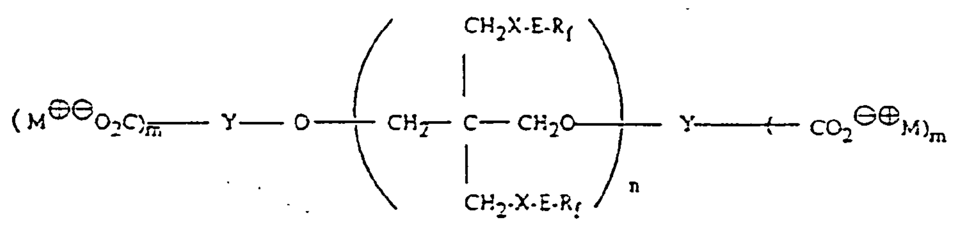

(I),

dans laquelle
$R_f$ représente un groupe fluoroaliphatique hydrophobe, oléophobe, comportant jusqu'à 20 atomes de carbone ;
E représente un groupe alkylène comportant jusqu'à 10 atomes de carbone, éventuellement interrompu par au plus trois groupes incluant -NR-, -O-, -S-, $-SO_2-$, $-O_2C-$, -CONR- et $-SO_2NR-$ ;
X représente -NR-, -O-, -S- ou $-SO_2-$ ;
Y représente indépendamment un atome d'hydrogène si l'un des m vaut zéro, ou Y représente un groupe alkylène à chaîne droite ou ramifiée ou un radical trivalent ou tétravalent, comportant jusqu'à 10 atomes de carbone, ou un groupe alkylène à chaîne droite ou ramifiée ou un radical trivalent ou tétravalent, comportant jusqu'à 10 atomes de carbone et interrompu par un groupe choisi dans l'ensemble que constituent -O-, -S- et -CH=CH-, ou encore un groupe carbonyl-alkylène, arylène ou carbonyl-arylène ;
R représente indépendamment un atome d'hydrogène, un groupe alkyle ou un groupe hydroxyalkyle comportant de 1 à 6 atomes de carbone ;
n représente un nombre entier valant de 1 à 3 ;
m vaut indépendamment 0, 1, 2 ou 3, avec la réserve que m ne peut pas valoir 0 dans les deux cas ;
M représente indépendamment un ion hydrogène, un ion de métal alcalin, un ion ammonium ou un ion organo-ammonium.

2. Composé conforme à la revendication 1, dans lequel ledit $R_f$ représente un groupe perfluoroalkyle comportant de 2 à 14 atomes de carbone ou un groupe perfluoroalkyle comportant de 2 à 6 atomes de carbone et substitué par un groupe perfluoroalcoxy comportant de 2 à 6 atomes de carbone.

3. Composé conforme à la revendication 1, dans lequel ledit $R_f$ représente un groupe perfluoroalkyle comportant de 6 à 14 atomes de carbone.

4. Composé conforme à la revendication 1, dans lequel ledit E représente un groupe alkylène à chaîne droite ou ramifiée, comportant de 2 à 6 atomes de carbone, éventuellement interrompu par un groupe choisi dans l'ensemble constitué par -NR-, -O-, -S -, $-SO_2-$, $-O_2C-$, -CONR- et $-SO_2NR-$.

5. Composé conforme à la revendication 1, dans lequel ledit E représente un groupe éthylène.

6. Composé conforme à la revendication 1, dans lequel ledit X représente -O-, -S- ou $-SO_2-$.

7. Composé conforme à la revendication 1, dans lequel ledit X représente -S- ou $-SO_2-$.

8. Composé conforme à la revendication 1, dans lequel ledit Y représente indépendamment un atome d'hydrogène ou un groupe alkylène à chaîne droite ou ramifiée ou un radical trivalent, comportant jusqu'à 6 atomes de carbone et interrompu par -O-, -S- ou -CH=CH-, ou encore un groupe carbonyl-alkylène, arylène ou carbonyl-arylène.

9. Composé conforme à la revendication 8, dans lequel ledit groupe alkylène ou radical trivalent est interrompu par un groupe carbonyl-alkylène.

10. Composé conforme à la revendication 1, dans lequel ledit R représente indépendamment un atome d'hydrogène, un groupe alkyle comportant de 1 à 7 atomes de carbone ou un groupe hydroxyalkyle comportant de 1 à 6 atomes de carbone.

**11.** Composé conforme à la revendication 1, dans lequel ledit R représente indépendamment un atome d'hydrogène, un groupe méthyle ou un groupe hydroxyéthyle.

**12.** Composé conforme à la revendication 1, dans lequel ledit n vaut 1 ou 2 et m vaut indépendamment de 1 à 3.

**13.** Composé conforme à la revendication 1, dans lequel ledit M représente un ion organo-ammonium.

**14.** Composé conforme à la revendication 1, dans lequel ledit $R_f$ représente un groupe perfluoroalkyle comportant de 6 à 14 atomes de carbone, E représente un chaînon alkylène comportant au plus 10 atomes de carbone, X représente -S-, Y représente un groupe carbonylalkylène, m vaut 1 et M représente un ion organo-ammonium.

**15.** Composé conforme à la revendication 1, dans lequel ledit $R_f$ représente un groupe perfluoroalkyle comportant de 6 à 12 atomes de carbone, E représente un chaînon éthylène, X représente $-SO_2-$, Y représente un groupe alkylène, m vaut 1 et M représente un ion organo-ammonium.

**16.** Composé conforme à la revendication 1, dans lequel ledit $R_f$ représente un groupe perfluoroalkyle comportant de 2 à 14 atomes de carbone ou un groupe perfluoroalkyle comportant de 2 à 6 atomes de carbone et substitué par un groupe perfluoroalkoxy comportant de 2 à 6 atomes de carbone, E représente un chaînon alkylène comportant de 2 à 6 atomes de carbone, $-CONHCH_2CH_2-$, $-CH_2CH_2N(CH_3)CH_2-$ $CH_2-$, $-CH_2CH_2SO_2NCH_2CH_2-$ ou $-SO_2NCH_2CH_2-$, X représente -S- ou $-SO_2-$, m vaut 1 ou 2 et M représente un ion organo-ammonium.

**17.** Composition pour traitement d'encollage, comportant au moins un composé conforme à la revendication 1.

**18.** Compositions pour traitement d'encollage, comportant d'environ 0,0005 % à environ 0,2 % en poids d'au moins un composé conforme à la revendication 1, de 0 à environ 0,05 % en poids d'un émulsifiant, de 0 à environ 5 % en poids d'un auxiliaire répulsif, d'un fongicide, d'une charge, d'un agent bactériostatique, d'un agent colorant ou d'un adjuvant de conditionnement de surface, et de 0 à environ 10 % d'un agent d'encollage, le reste étant de l'eau.

**19.** Utilisation des composés de formule I et conformes à la revendication 1, pour traiter des surfaces pour leur conférer un caractère oléophobe et hydrophobe.